# EUROPEAN PATENT APPLICATION

(11) **EP 4 440 155 A1**
(43) Date of publication of application: **02.10.2024**
(21) Application number: 23165779.2
(22) Date of filing: 31.03.2023
(51) Int. Cl.: H04R 25/00, A61N 1/05, A61N 1/36, A61N 1/372, H04B 5/00, H04L 65/1069, H04L 65/65, H04W 4/80

(54) **ACQUSITION SEQUENCES OF BINAURAL HEARING SYSTEMS COMPRISING HEARING IMPLANTS**

(71) Applicant: GN Hearing A/S, 2750 Ballerup (DK)
(72) Inventor: LØNGAA, Michael, 2750 Ballerup (DK)
(74) Representative: Guardian IP Consulting I/S

(57) **Abstract**

The present disclosure relates to acquisition sequences of binaural hearing systems and corresponding binaural hearing systems. The binaural hearing systems may comprise first and second hearing devices, for example head-wearable devices at a user's ears, and first and second hearing implants.

## Description

The present disclosure relates to acquisition sequences of binaural hearing systems and corresponding binaural hearing systems. The binaural hearing systems may comprise first and second hearing devices, for example head-wearable devices at a user's ears, and first and second hearing implants.

### BACKGROUND OF THE INVENTION

Binaural hearing systems that comprise a pair of hearing implants, such as cochlea hearing implants, may comprise at least four separate devices that are wirelessly connected via at least three bidirectional wireless communication links during normal operation. A first bidirectional wireless communication link must be connected between a first hearing device, typically arranged at, in or on a user's ear, and a first hearing implant such as a cochlea implant. A second bidirectional wireless communication link must be connected between a second hearing device, typically arranged at, in or on the user's opposite ear, and a second hearing implant. Finally, a third bidirectional wireless communication link must be connected between the first and second hearing devices, i.e. a bilateral link, to allow the devices at opposite sides of the user's head to communicate data for example digital audio signals to enable sophisticated binaural processing algorithms and/or synchronize other functions of the first and second hearing devices.

There is need for a hearing systems, that for example use a common communication protocol between devices and implants, and support rapid and energy efficient acquisition of each of the first and second bidirectional wireless communication links as well as the bilateral link at start-up of the binaural hearing system. There is also a need for energy efficient operation of the first and second hearing devices and the first and second hearing implants during normal operation of the binaural hearing system. Each of the first and second hearing devices and the first and second hearing implants is typically a relatively small device energized by power sources with limited capacity like rechargeable batteries.

### SUMMARY OF THE INVENTION

A first aspect of the invention relates to a binaural hearing system comprising:
a first hearing device, a second hearing device, a first hearing implant and a second hearing implant; wherein
the first hearing device is connectable to the first hearing implant via a first bidirectional wireless communication link for exchange of first ipsilateral data packets,
the second hearing device is connectable to the second hearing implant via a second bidirectional wireless communication link for exchange of second ipsilateral data packets,
a bilateral bidirectional wireless communication link is connectable between the first hearing device and second hearing device for exchange of bilateral data packets. The bilateral bidirectional wireless communication link and said first and second bidirectional wireless communication links are preferably configured to operate in accordance with a common communication protocol. The common communication protocol comprises a plurality of consecutive frames each comprising a plurality of time slots and an acquisition sequence. The latter comprises:
   acquire the bilateral bidirectional wireless communication link using at least a first processing unit of the first hearing device and a second processing unit of the second hearing device,
   acquire the first bidirectional wireless communication link in response to acquisition of the bilateral bidirectional wireless communication link using at least the first processing unit of the first hearing device,
   acquire the second bidirectional wireless communication link in response to acquisition of the bilateral bidirectional wireless communication link using at least the second processing unit of the second hearing device.

After a successful acquisition sequence of the binaural hearing system the latter preferably enters a normal operation mode where the first hearing device and the first hearing implant are wirelessly connected through the first bidirectional wireless communication link. The second hearing device and the second hearing implant are likewise wirelessly connected through the second bidirectional wireless communication link during normal operation. Finally, the first hearing device and the second hearing device are wirelessly connected through the bilateral bidirectional wireless communication link during normal operation. The common communication protocol may be proprietary and designed for minimal power consumption since each of the first and second hearing devices and each of the first and second hearing implants typically are relatively small battery powered devices or otherwise energized by a power source with limited capacity.

One of the first hearing device and second hearing device is preferably configured as master and the other one as slave for the purpose of acquiring and operating the bilateral bidirectional wireless communication link using their respective first and second processing units as discussed in additional detail below with reference to the appended drawings.

Each of the first and second hearing devices may comprise a head-wearable housing shaped and sized similarly to a traditional hearing aid for example of so-called BTE, ITE, ITC, CIC or RIC types of housings. The housings may be shaped and sized for placement at, or in, a user's left or right ear for example shaped and sized for placement behind the user's left and right ear pinna. The first and second hearing implants may be configured for placement at respective sides of the user's skull and configured to supply respective nerve stimulus signals to the user's left and right hearing nerves via implanted electrode arrays.

Each of the first and second bidirectional wireless communication links and the bilateral bidirectional wireless communication link may be based on near-field magnetic coupling, such as NFMI, using respective magnetic coil antennas mounted in the first and second hearing devices. Each of the first and second bidirectional wireless communication links and the bilateral bidirectional wireless communication link may for example use a carrier frequency between 5 and 50 MHz as discussed in additional detail below with reference to the appended drawings.

According to one embodiment of the binaural hearing system at least a subset of the first and second ipsilateral data packets comprises respective digital audio data such as real-time digital audio signals; and/or at least a subset of the bilateral data packets comprises respective digital audio data such as real-time digital audio signals as discussed in additional detail below with reference to the appended drawings.

Each of the first processing unit and second processing unit may comprise a digital signal processor (DSP) and/or a microprocessor such as a software programmable DSP or a software programmable microprocessor. The software programmable DSP or microprocessor may be configured to execute plurality of program instructions configured to implement at least parts of the respective acquisitions of the first and second bidirectional wireless communication links and the bilateral bidirectional wireless communication link in accordance with the common communication protocol. Each of the first processing unit and second processing unit may comprise a dedicated digital state machine configured to handle certain steps of the acquisitions of the first and second bidirectional wireless communication links and the bilateral bidirectional wireless communication link in accordance with the common communication protocol.

According to an embodiment of the common communication protocol and corresponding binaural hearing system the acquisition of the bilateral bidirectional wireless communication link comprises:
- transmit a first synchronization marker from the first processing unit to the second processing unit in a first time slot at the first processing unit,
- monitor the plurality of time slots at the second processing unit for the first synchronization marker,
- slide the plurality of time slots at the second processing unit with predetermined time steps from frame to frame of the plurality of consecutive frames using a wrap-around scheme,
- detect the first synchronization marker at the second processing unit,
- transmit an acknowledge message from the second processing unit to the first processing unit in a second time slot at the first processing unit,
- synchronize the plurality of time slots at the second processing unit with the plurality of time slots at the first processing unit based on the first synchronization marker,
- complete the acquisition of the bilateral bidirectional wireless communication link,
- start the exchange of the bilateral data packets through the bilateral bidirectional wireless communication link in the first and second times slots of the consecutive frames.

A "time slot" is the shortest time division of the bilateral bidirectional wireless communication link and first and second bidirectional wireless communication links as defined by the common communication protocol. One data packet may for example be transmitted in one time slot.

A "frame" is a unit of data that comprises a predefined number of time slots as defined by a communication protocol.

"Connectable" shall mean that the wireless communication link in question is configured to establish a wireless connection between specified devices after acquisition of the wireless communication link in question.

A "bidirectional wireless communication link" is a wireless communication link that supports transmission of data packets or data messages from a first device to a second device and transmission of data packets or data messages from the second device to the first device.

"Ipsilateral data packets" are data packets exchanged between a hearing device and a hearing implant arranged at the same side of a user's head.

A "hearing implant" shall mean that portion of a cochlear implant that is located inside the user's skull.

According to an embodiment of the common communication protocol and corresponding binaural hearing system the acquisition of the first bidirectional wireless communication link comprises:
- transmit a second synchronization marker from the first processing unit to a first implant processor of the first hearing implant in a third time slot at the first processing unit,
- monitor the plurality of time slots at the first implant processor for receipt of the second synchronization marker,
- sliding the plurality of time slots at the first implant processor with predetermined time steps from frame to frame of the consecutive frames using a wrap-around scheme,
- detect the second synchronization marker at the first implant processor,
- synchronize the plurality of time slots at first implant processor with the plurality of time slots at the first processing unit based on the second synchronization marker,
- complete the acquisition of the first bidirectional wireless communication link,-transmit an acknowledge message from the first implant processor to the first processing unit in a fourth time slot at the first processing unit; and
the acquisition of the second bidirectional wireless communication link comprises:
- transmit a third synchronization marker from the second processing unit to a second implant processor of the second hearing implant in a third time slot at the second processing unit,
- monitor the plurality of time slots, at the second implant processor for receipt of the third synchronization marker,
- sliding the plurality of time slots at the second implant processor with predetermined time steps from frame to frame of the plurality of consecutive frames using a wrap-around scheme,
- detect the third synchronization marker at the second implant processor,
- synchronize the plurality of time slots at second implant processor with the plurality of time slots at the second processing unit based on the third synchronization marker,-transmit an acknowledge message from the second implant processor to the second processing unit in a fourth time slot at the second processing unit,
- complete the acquisition of the second bidirectional wireless communication link; and complete the acquisition sequence and enter normal operation of the binaural hearing system.

Each frame of the plurality of consecutive time frames may comprise at least four time slots such as four overlapping time slots. The length of each of the at least four time slots may be identical as discussed in additional detail below with reference to the appended drawings.

In one embodiment each of the predetermined time steps for the sliding of the plurality of time slots is less than 5 %, for example less than 1 %, of a length of one time slot of the plurality of time slots of the frame as discussed in additional detail below with reference to the appended drawings.

According to an embodiment of the common communication protocol and corresponding binaural hearing system, the first synchronization marker comprises a unique pair ID for pairing the first hearing device and the second hearing device; and
- the second synchronization marker comprises a unique pair ID for pairing the first hearing device and first hearing implant; and
- the third synchronization marker comprises a unique pair ID for pairing the second hearing device and the second hearing implant.

The first synchronization marker transmitted from the first processing unit to the second processing unit during acquisition of the bilateral bidirectional wireless communication link may for example comprise a first unique pair ID. The second processing unit is configured to:
- compare the first unique pair ID of the synchronization marker with a pre-stored unique pair ID,
- ignore the first synchronization marker if the first unique pair ID does not match the pre-stored unique pair ID; and
- transmit the acknowledge message if the pre-stored unique pair ID matches the first unique pair ID of the first synchronization marker. Certain embodiments of the binaural hearing system utilize three unique pair IDs, i.e. two additional unique pair IDs in addition to the first unique pair ID, in the above-mentioned pairings of the first hearing device and the first hearing implant, the first hearing device and the second hearing device and the second hearing device and the second hearing implant. This embodiment is favourable because the use of unique pair IDs of the first, second and third synchronization markers avoids erroneous pairings of devices of the binaural hearing system during the acquisition sequence as discussed in further detail below with reference of the appended drawings.

The synchronization marker may at least comprise:
- a predetermined binary pattern,
- a time slot indicator for indicating which time slot of the plurality of time slots at the first processing unit that holds the synchronization marker, and optionally and error-detecting code.

The common communication protocol may comprise a plurality of super frames each comprising a plurality of individual frames, such as between 4 and 32 individual frames, of the plurality of consecutive frames. In the latter embodiment, the second processing unit is preferably configured to determine an offset in frames between consecutive super frames,
- transmit the offset to the first processing unit via the bilateral bidirectional wireless communication link while the first processing unit is configured to read the offset and adjust timing of the super frames at the first processing unit to align super frames at the first and second processing units.

According to one embodiment of the binaural hearing system, the acquisition sequence comprises:
- maintain connection through the bilateral bidirectional wireless communication link during the acquisition of the first bidirectional wireless communication link and during the acquisition of the second bidirectional wireless communication link.

A second aspect of the invention relates to an acquisition sequence, e.g. an computer-implemented acquisition method, in accordance with the common communication protocol for acquiring respective connections between a first hearing device and a first hearing implant, a second hearing device and a second hearing implant and the first hearing device and the second hearing device. The acquisition sequence comprising:
- acquire a bilateral bidirectional wireless communication link between the first hearing device and the second hearing device using at least a first processing unit of the first hearing device and a second processing unit of the second hearing device,
- exchange bilateral data packets between the first hearing device and the second hearing device,
- acquire a first bidirectional wireless communication link between the first hearing device and the first hearing implant, in response to acquisition of the bilateral bidirectional wireless communication link, using at least the first processing unit of the first hearing device,
- exchange first ipsilateral data packets between the first hearing device and the first hearing implant,
- acquire a second bidirectional wireless communication link between the second hearing device and second hearing implant, in response to acquisition of the bilateral bidirectional wireless communication link, using at least the second processing unit of the second hearing device
- exchange second ipsilateral data packets between the second hearing device and the second hearing implant;
- enter normal operation of the binaural hearing system.

One embodiment of the acquisition sequence comprises:
- exchange the bilateral data packets in first and second time slots at the first processing unit of respective frames of the plurality of consecutive frames,
- exchange the first ipsilateral data packets in third and fourth time slots at the first processing unit of the respective frames of the plurality of consecutive frames,
- exchange the second ipsilateral data packets using in the third and fourth time slots at the second processing unit of respective frames of the plurality of consecutive frames.

### BRIEF DESCRIPTION OF THE DRAWINGS

In the following exemplary embodiments of the invention are described in more detail with reference to the appended drawings, wherein:
FIG. 1 schematically illustrates an exemplary binaural hearing system in accordance with the invention mounted on user's head,
FIG. 2 shows respective schematic block diagrams of first and second hearing devices and associated first and second hearing implants of the exemplary binaural hearing system,
FIG. 3 schematically illustrates transmission of a first synchronization marker from the first hearing device to the second hearing device during acquisition of a bilateral bidirectional wireless communication link according to embodiments of the exemplary binaural hearing system,
FIG. 4 schematically illustrates exchange of bilateral data packets between the first hearing device and the second hearing device after the acquisition of the bilateral bidirectional wireless communication link according to embodiments of the exemplary binaural hearing system,
FIG. 5 schematically illustrates acquisition of the first bidirectional wireless communication link and acquisition of the second bidirectional wireless communication link in accordance with embodiments of the exemplary binaural hearing system,
FIG. 6 schematically illustrates exchange of first and second ipsilateral data packets and exchange of bilateral data packets during normal operation, i.e. payload mode, of the binaural hearing system after completion of an acquisition sequence in accordance with embodiments of the exemplary binaural hearing system,
FIG. 7 illustrates features of an exemplary synchronization marker utilized during the acquisition of the bilateral bidirectional wireless communication link in accordance with the embodiments of the exemplary binaural hearing system,
FIG. 8 illustrates exemplary super-frame structures each comprising a plurality of frames of the ipsilateral data packets and bilateral data packets in accordance with the embodiments of the exemplary binaural hearing system,
FIG. 9 and FIG. 10 are flow charts illustrating respective processing steps carried out by the first and second processing units of the first and second hearing devices, respectively, during acquisition of the bilateral bidirectional wireless communication link according to embodiments of the exemplary binaural hearing system.

### DETAILED DESCRIPTION OF EMBODIMENTS

In the following various exemplary embodiments of the present hearing system are described with reference to the appended drawings. The skilled person will understand that the accompanying drawings are schematic and therefore merely show details which are essential to understanding of the invention. Other details may have been left out or simplified for brevity and clarity. Like reference numerals refer to like elements throughout the drawings. Like elements will, thus, not necessarily be described in detail with respect to each drawing.

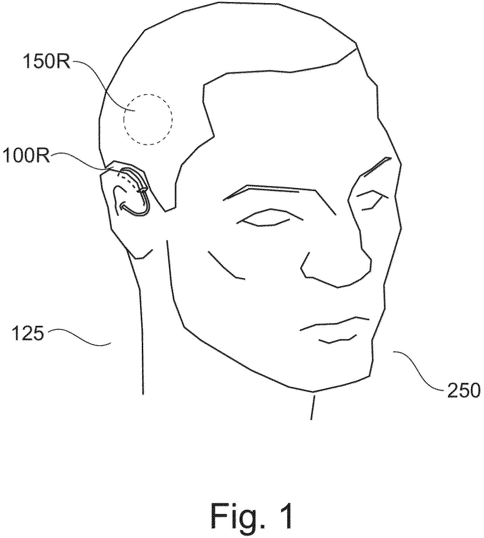

FIG. 1 schematically illustrates a first hearing device 100L and a first hearing implant 150L of an exemplary binaural hearing system 125. The binaural hearing system 125 further comprises a second hearing device 100L and a second hearing implant 150L both arranged at the opposite side of the user's head 250 and therefore not visible. The first hearing implant 150L is adapted for surgical implantation in the user's skull and therefore invisible from the outside when implanted. The second hearing implant 150R is arranged in a corresponding manner at the opposite side of the user's head 250.

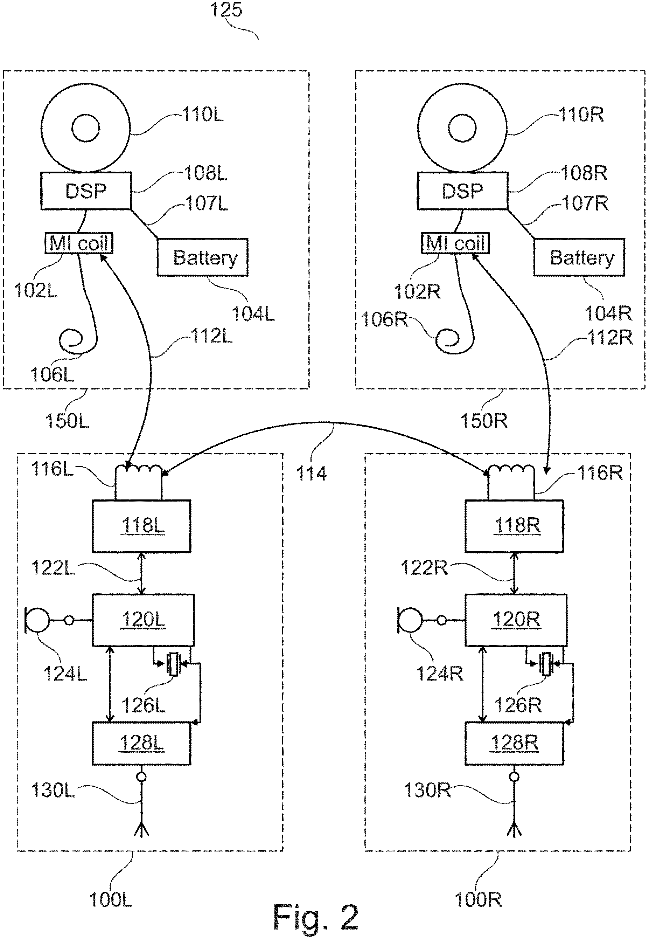

FIG. 2 is a block diagram of the binaural hearing system 125. The first hearing device 100L is connectable to the first hearing implant 150L via a first bidirectional wireless communication link 112L ("first link") for exchange of first ipsilateral data packets. The second hearing device 100L is likewise connectable to the second hearing implant 150L via a second bidirectional wireless communication link 112L ("second link") for exchange of second ipsilateral data packets.

The binaural hearing system 125 further comprises a bilateral bidirectional wireless communication link 114 ("bilateral link", or "third link") that is connectable between the first hearing device 100L and second hearing device 100R for exchange of bilateral data packets. The first hearing device 100L and second hearing device 100R are preferably configured to use the respective magnetic coil antennas 116L, 116R for wireless communication with the first hearing implant 150L and second hearing implant 150R, respectively. The bilateral link 114, first link 112L and second link 112R are preferably configured to utilize, i.e. operate in accordance with, a common data communication protocol. The common data communication protocol comprises a plurality of consecutive time frames that each comprises a plurality of time slots holding the first and second ipsilateral data packets and the bilateral data packets. The respective pluralities of first and second ipsilateral data packets and bilateral data packets are structured, transmitted and received in accordance with the common data communication protocol as discussed in further detail below.

The skilled person will understand that certain types of data packets such as synchronization markers and acknowledge messages may be wirelessly transmitted between the first hearing device 100L and second hearing device 100R, between the first hearing device 100L and the first hearing implant 150L and between the second hearing device 100R second hearing implant 150R before the respective acquisitions of the bilateral link 114, first link 112L and second link 112R.

The bilateral data packets exchanged by the bilateral link 114 may comprise digital audio samples and control information transmitted by respective ones of the first and second hearing devices 100L, 100R. Each of the first and second hearing devices 100L, 100R may transmit digital audio samples and control data to the first and second hearing implant 150L, 150R, respectively. Each of the first and second hearing implants 150L, 150R, preferably utilizing the first and second control units, e.g. implant processor, 108L, 108R, respectively, may additionally be configured to transmit respective control data to the first and second hearing devices 100L, 100R via the first link 112L and second link 112R. In the latter embodiment both of the first link 112L and second link 112R are adapted for bidirectional data transfer.

Each of the first link 112L, second link 112R and bilateral link 114 may in certain embodiments of the invention be based on, or comprise, near-field magnetic coupling, such as NFMI links. The first link 112L may accordingly comprise a magnetic coil antenna 116R of the first hearing device 100L and a magnetic coil antenna 102R of the first hearing implant 150L. The second link 112R may likewise comprise a magnetic coil antenna 116L of the second hearing device 100L and a magnetic coil antenna 102L of the second hearing implant 100L. Each of first link 112L, second link 112R and bilateral link 114 may for example utilize a carrier frequency between 5 MHz and 50 MHz, such as between 9 MHz and 27 MHz for transmissions of the first and second ipsilateral data packets and the bilateral data packets.

The first hearing implant 150L comprises the first control unit 108L that is connected to the magnetic coil antenna 102L for receipt and processing of the first ipsilateral data packets transmitted by the first hearing device 100L. The first control unit 108L is further configured to transmit the first ipsilateral data packets to the first hearing device 100L via the first link 112L. In some embodiments, a first processing unit 108L of the first hearing implant 150L may comprise a digital signal processor (DSP) and/or a microprocessor. Certain embodiments of the first hearing implant 150L may comprise a rechargeable battery assembly 104L. The rechargeable battery assembly 104L is configured for receiving electrical power from a receiver (Rx) charging coil 110L during dedicated recharging operations or sessions. The receiver (Rx) charging coil 110L may be energized by an appropriately positioned external, i.e. outside the user's skull, transmitter (Tx) charging coil (not shown) of a charging device (not shown) during the dedicated recharging sessions. This allows wireless transfer of power to the rechargeable battery assembly 104L. The rechargeable battery assembly 104L is preferably coupled to a power supply input of an implant processor 108L to energize the latter as schematically indicated by a power line or wire 107L. The implant processor 108L may comprise a microprocessor for example software programmable microprocessor.

The first hearing implant 150L further comprises an electrode array 106L for insertion into a cochlea of the user during implantation. The electrode array 106L may be electrically coupled to the first processing unit 108L that supplies suitable electrode stimuli signals to the electrode array 106L to stimulate the user's cochlea nerve. The skilled person will understand that these electrode stimuli signals may be generated by the first processing unit 108L and derived by the latter based on the first ipsilateral data packets, in particular digital audio signals or data embedded or held in the first ipsilateral data packets. The skilled person will appreciate that corresponding functions, structures and features of the second hearing implant 150R may be largely identical to those of the first hearing implant 150L.

The block diagram on FIG. 2 further illustrates an exemplary embodiment of the first hearing device 100L. The first hearing device 100L comprises the magnetic coil antenna 116L which is electrically connected to a transceiver 118L. The transceiver 118R is configured to repeatedly switch between Tx and Rx modes to modulate and demodulate incoming and outgoing data of the first ipsilateral data packets and bilateral data packets. The transceiver 118L may be configured to convert the first ipsilateral data packets and bilateral data packets to a format understood by a first processing unit 120L of the first hearing device 100L. In some embodiments, the first processing unit 120L may comprise a digital signal processor (DSP) and/or a microprocessor such as a software programmable DSP or a microprocessor.

The first hearing device 100L further comprises one or more microphones 124L that are coupled to an appropriate audio interface of the first processing unit 120L. The first processing unit 120L may be configured to execute a suitable operating system. The operating system may be configured to manage various hardware and software resources of the first hearing device 100L such as handling of the common communication protocol, computation of monaurally or bilaterally beamformed microphone signals, hearing loss compensation processing of the microphone signal(s), the first wireless data communication interface 118L, certain memory resources etc. The operating system may schedule tasks for efficient use of hearing device resources and may further include accounting software for cost allocation, including power consumption, processor time, memory locations, wireless transmissions, and other resources. The operating system may be stored in and retrieved from a non-volatile memory (not shown), e.g. flash memory or EEPROM, of the processing unit 120L.

Each of the first and second hearing devices 100L, 100R may comprise a housing of a type that is well-known from the hearing aid industry like so-called BTE, ITE, ITC, CIC or RIC housing types. These housing types are shaped and sized for placement at, or in, the user's ear. The first hearing device 100L further comprises a system clock generator 126L that is configured to supply system clock signals to various digital logic circuits and components of the device 100L including the first processing unit 120R as schematically illustrated. A nominal value of a clock frequency of the system clock generator 126R may lie between 2 MHz and 64 MHz such as between 10 MHz and 50 MHz. The first processing unit 120R may be configured to derive respective lengths of certain time slots and time frames of the common communication protocol of the first link 112L and the bilateral link 114 as discussed in additional detail below.

The first hearing device 100L may additionally comprises a second, optional, wireless communication interface 128L and RF antennal 130L configured to jointly communicate through a second wireless communication link (not shown). The second wireless communication link and interface may be configured to operate in the 2.4 GHz industrial scientific medical (ISM) band and may be compliant with a Bluetooth LE standard. This second wireless communication link may provide convenient data connectivity to various types of portable communication devices like smartphones, mobile phones, tablets and personal computers etc. due to the industry standard compatible nature of the second wireless communication link. Various types of control data and audio data may be transmitted from the portable communication device to the first hearing device 100L and vice versa. The second hearing device 100L may for the same purpose comprise a similar optional second wireless communication interface 128L and RF antenna 130L as illustrated.

In the following disclosure the first hearing device 100L of the binaural hearing system 125 is assigned as master during execution of the common communication protocol while the second hearing device 100R is configured as a slave. The skilled person will understand that the system clock signal of the master processing unit 120L may control timing of transmissions of respective data packets through the first link 112L, second link 112R and bilateral link 114 at least after the acquisitions of the first link 112L, second link 112R and bilateral link 114.

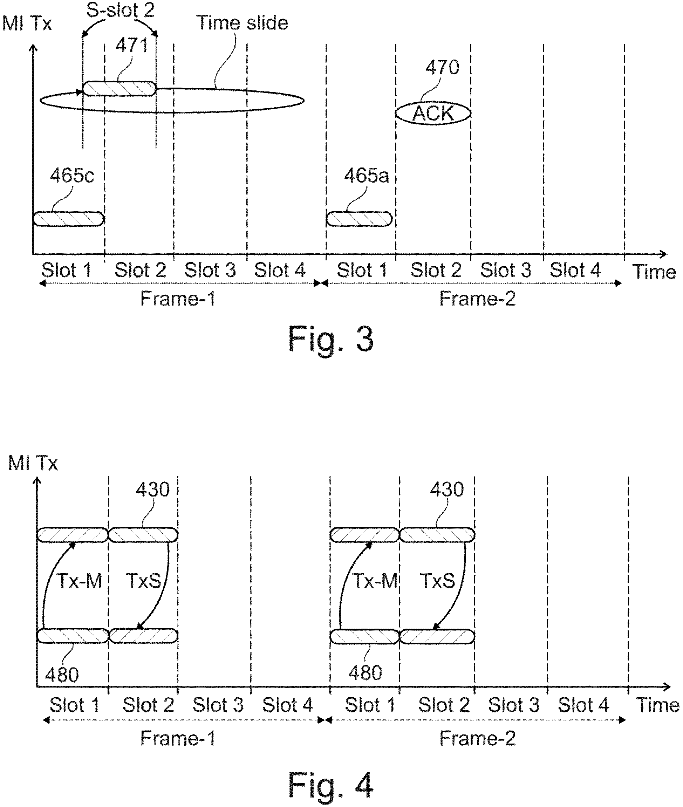

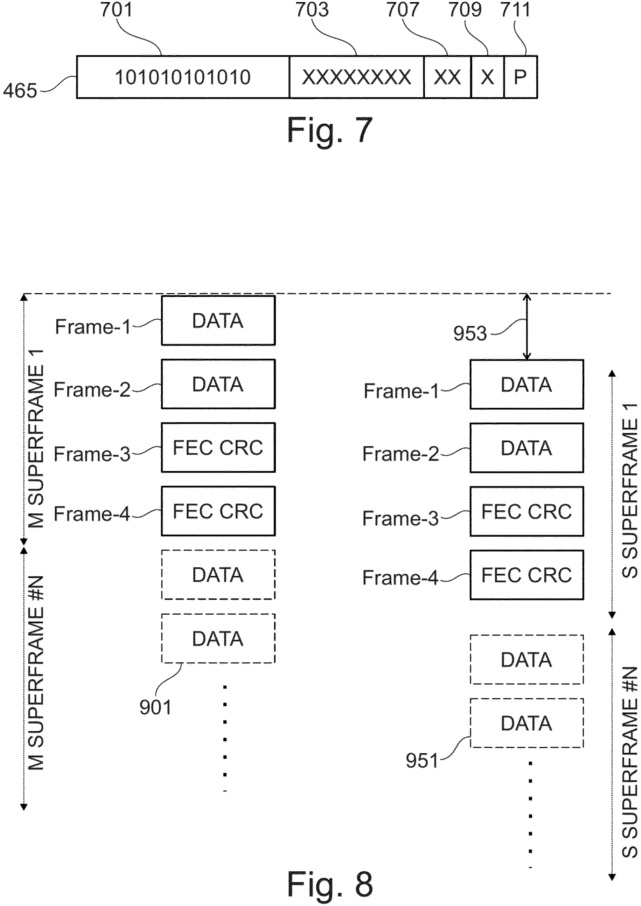

FIG. 3 schematically illustrates transmission of a first synchronization marker 465c from the master processing unit 120L of the first or master hearing device 100L to the second or slave hearing device 100R during acquisition of the bilateral link 114 according to embodiments of the common communication protocol of the exemplary binaural hearing system 125. During the acquisition of the bilateral link 114, the master processing unit 120L operates as a master device towards the second hearing device 100R. The master processing unit 120L may start to repeatedly transmit the first synchronization marker 465c to the second hearing device 100R. using one of a plurality of time slots, e.g. time slots 1-4, of each frame, Frame-1, Frame-2 etc., of a plurality of consecutive frames. The indicated time slots 1-4 are referenced to the master processing unit 120L. Time slot 1, at the master processing unit 120L, is selected for transmission of the first synchronization marker 465c in the present example as illustrated and made in accordance with the common communication protocol. The second hearing device 100R resides concurrently in a receipt mode such that the second, or slave, processing unit 102R of the slave hearing device 100R monitors the bilateral link 114 to detect receipt of the first synchronization marker 465c. However, the respective time slots 1-4 at the master processing unit 120L and the first hearing implant 150L are most likely more or less unaligned or non-synchronous at the current acquisition step of the acquisition sequence caused by free-running clock generators and system clock signals at the master processing unit 120L and the slave processing unit 120R. As illustrated by FIG. 3 where the slave hearing device 100R searches for the first synchronization marker 465c in a time slot 471 at the slave hearing device 100R which exemplary time slot 471 is shifted or misaligned, as indicated by the S-slot-2 arrow, with about one-fourth of the length of one time slot relative to the time slots at the master processing unit 120L. This misalignment of time slots prevents the slave hearing device 100R from detecting the first synchronization marker 465c because the latter extends across two adjacent time slots at the slave hearing device 100R. The slave processing unit 120R advances or slides its time slots 1-4, such as the exemplary time slot 471, with predetermined time steps between frames of the plurality of consecutive frames using a wrap-around scheme as schematically indicated by "Time slide" arrow. The wrap-around scheme means that that the sliding of the time slots slave processing unit 120R, such as S-slot 2, jumps back to time slot 1 when the end of time slot 4 is reached by the sliding operation. The predetermined time step for the slot sliding is preferably much shorter than a length of one time slot such as less 5 % or less than 1 % of the length of one time slot to gradually align the time slots 1-4 at the slave processing unit 120R with the time slots 1-4 at the master processing unit 120L. The slave processing unit 120R may continue to slide its time slots 1-4 by a wrap-around scheme until the latter time slots are sufficiently aligned with the time slots 1-4 at the master processing unit 120L to detect receipt of the first synchronization marker 465c at the slave processing unit 120R. The slave processing unit 120R may check the unique pair ID of the received first synchronization marker to ensure it is transmitted from the paired device, i.e. the master hearing device 100L in the present situation. The search for the first synchronization marker 465c by the slave processing unit 120R may optionally comprise recognition of a predetermined search sequence 701 (FIG. 7) as described in further detail below.

The slave processing unit 120R may be configured to ignore the first synchronization marker 465c if it is not transmitted by the paired device, i.e. the unique pair ID of the received synchronization marker does not match that of the first synchronization marker 465c. In the latter situation slave processing unit 120R may thereafter continue the search for the first synchronization marker 465a. When the unique pair ID of the received synchronization marker is a match to that of the first synchronization marker 465c, the slave processing unit 120R may respond by detecting the time slot, e.g. time slot 1, at the master processing unit 120L in which the first synchronization marker 465c was transmitted by a slot indicator bit or field of the first synchronization marker as discussed in further detail below. The slave processing unit 120R may, based on that time slot identification, align, i.e. synchronize, the time slots at the slave processing unit 120R with the corresponding time slots at the master processing unit 120L of the first hearing device 100L. Hence, synchronize data packet exchange or communication between the slave processing unit 120R and the master processing unit 120L through the bilateral link 114.

The slave processing unit 120R thereafter shifts to a transmission mode and transmits an acknowledge message 470 (ACK) to the master processing unit 120L in time slot 2 at the master processing unit 120L as schematically illustrated by FIG. 3. Thereafter, the master processing unit 120L may respond by completing or terminating the acquisition of the bilateral link 114 because the latter is now appropriately connected between the master processing unit 120 and the slave processing unit 120R in accordance with exemplary embodiments of the common communication protocol of the binaural hearing system 125. The master processing unit 120L and the slave processing unit 120R are accordingly ready to exchange the bilateral data packets 430, 480 using time slots 1 and 2 of the plurality of consecutive frames as illustrated by FIG. 4. In time slot 1 of frame 1, the master processing unit 120L transmits a first bilateral data packet 430 to the slave processing unit 120R which resides in a receipt mode or state during time slot 1. In time slot 2 of frame 1, the slave processing unit 120R transmits a second bilateral data packet 480 to the master processing unit 120L which now has switched to a receipt mode or state during time slot 2.

The skilled person will understand that other embodiments of the common communication protocol may specify more than four time slots per frame such as between 5 and 16 time slots. Irrespective of the actual number of time slots, such as at least four time slots, these are preferably non-overlapping time slots. Each time slot may have a length between 24 µs and 384 µs for example depending a number of bits of at least some of the exchanged data packets. The length of each of the time slots may be identical. Each of the first ipsilateral data packets 455, 460 and the second ipsilateral data packets 405, 410 may comprise identical number of bits for example between 4 bits and 96 bits such as between 6 bits and 24 bits. The number of bits in a particular data packet may depend on content type, i.e. digital audio signals, control information or a combination of both.

At least some of the bilateral data packets 430, 480 comprise digital audio signals and/or control information. The control information may be held in a packet header and the digital audio signals held in a packet payload. The digital audio signals may be perceptually encoded to reduce the amount of audio data in the messages for transmission. The respective digital audio signals of the bilateral data packets 430, 480 may be generated by, or derived, from respective microphone arrangements of the master and slave hearing devices 100L, 100R. These microphone arrangements may for example be integrated in respective housings of the master and slave hearing devices 100L,100R. The respective digital audio signals of the bilateral data packets 430, 480 may alternatively be derived from remote microphone arrangements wirelessly coupled to the master and slave hearing devices 100L, 100R. The sound may comprise speech, noise or any mixture thereof for example sound present in a user's external environment or sound streamed sound from an audio enabled portable device.

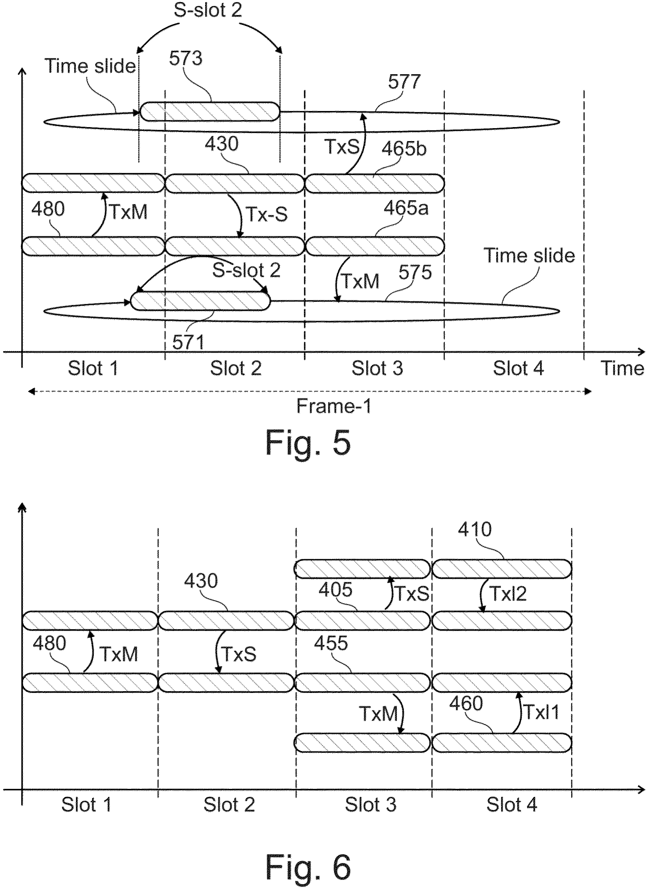

FIG. 5 schematically illustrates acquisition of the first link 112L and acquisition of the second link 112R in accordance with embodiments of the exemplary binaural hearing system 125. As outlined above the acquisition of the bilateral link 114 has been completed and in response the master processing unit 120L initiates the acquisition of the first link 112L and the acquisition of the second link 112R. FIG. 5 illustrates how the master processing unit 120L and the slave processing unit 120R exchange the bilateral data packets 430, 480 using time slots 1 and 2 of a frame. The master processing unit 120L may further be configured to repeatedly generate and transmit the second synchronization marker 465a to the first control unit 108L of the first hearing implant 150L using an unoccupied time slot of the frames, like time slot 3, at the master processing unit 120L. The first control unit 108L operates in its receipt mode and searches for the second synchronization marker 465a in a time slot 571 at the slave hearing device 100R. The exemplary time slot 571 is shifted or misaligned, as indicated by the S-slot-2 arrow, with about one-fourth of the length of one time slot relative to the time slots at the master processing unit 120L indicated on the x-axis. This misalignment of time slots prevents the first control unit 108L from detecting the second synchronization marker 465a for the reasons discussed above in connection with the first synchronization marker 465c. The first control unit 108L therefore advances or slides its time slots 1-4, such as the exemplary time slot 571, with predetermined time steps from frame to frame of the plurality of consecutive frames using a wrap-around scheme as schematically indicated by "Time slide" arrow 575. The predetermined time step is preferably shorter than the length of one time slot such as less 5 % or less than 1 % of the length of one time slot. The predetermined time step may be 250 ns for a slot length of about 48 µs. Following this scheme, the first control unit 108L is configured to gradually align the time slots 1-4 at the first control unit 108L with the time slots 1-4 at the master processing unit 120L. The first control unit 108L may continue to slide its time slots 1-4 until the latter time slots are sufficiently aligned with the time slots 1-4 at the master processing unit 120L to detect receipt of the second synchronization marker 465a at the first control unit 108L. The first control unit 108 may optionally proceed to check the unique pair ID of the received synchronization marker 465a to ensure it is transmitted from a paired device, i.e. the master hearing device 100L in the present situation. The first control unit 108L may be configured to ignore the synchronization marker if it is not transmitted by the paired device, i.e. the unique pair ID of the received synchronization marker does not match that of the second synchronization marker 465a. In the latter situation, the first control unit 108L may thereafter continue searching for the second synchronization marker 465a. When the unique pair ID of the received synchronization marker 465a is a match, the first control unit 108L may respond by reading the slot indicator 709 of the third synchronization marker 465b and align its time slots 1-4 at with the corresponding time slots at the master processing unit 120L to synchronize the ipsilateral data packet exchange between the first hearing implant 150L and the master hearing device 100L through the first link 112L. The first control unit 108L thereafter shifts to a transmission mode and transmit an acknowledge message (not shown) to the master processing unit 120L in the unoccupied time slot 4 at the master processing unit 120L.

The master processing unit 120L may respond to receipt of the acknowledge message by completing the acquisition of the first link 112L because the latter is now appropriately connected between the master hearing device 100L and the first hearing implant 150L in accordance with the exemplary embodiments of the common communication protocol of the binaural hearing system 125. The master hearing device 100L and the first implant 150L are accordingly ready to exchange the first ipsilateral data packets 455, 460 using time slots 3 and 4 of the plurality of consecutive frames as illustrated by FIG. 6. In time slot 3 of frames 1, the master hearing device 100L transmits, TxM, an ipsilateral bilateral data packet 455 to the first hearing implant 150L.

The first implant 150L resides in the receipt mode or state in time slot 3. In time slot 4 of frames 1, the first hearing implant 150L transmits, Txl1,, a second ipsilateral data packet 460 to the master processing unit 120L which now has switched to a receipt mode or state, from the previous transmit mode in time slot 3. Further in time slot 4 of the frames, the second hearing implant 150R transmits, Txl2, a second ipsilateral data packet 410 to the slave processing unit 120R which now has switched to a transmission mode or state from the previous receipt mode in time slot 3. The skilled person will appreciate that the acquisition of the second link 112R of the exemplary binaural hearing system 125 may follow a corresponding acquisition scheme of the first link 112R to create the wireless connection between the slave hearing device 100R and the second hearing implant 150R. Preferably, the slave processing unit 120R operates as a master device towards the second hearing implant 150R during acquisition. Briefly, the slave processing unit 120L may be configured to repeatedly generate and transmit the third synchronization marker 465b to the second control unit 108L of the second hearing implant 150R using an unoccupied time slot of the frames, like time slot 3, at the master processing unit 120L. The third synchronization marker has a different pair ID, than that of each of the first and second synchronization markers 465c, 465a and is preferably utilized by the slave processing unit 120R and the second hearing implant 150L for the acquisition of the second link 112R. The second control unit 108L advances or slides its time slots 1-4, such as the exemplary time slot 573, with predetermined time steps in the same manner as described in detail above in connection with the acquisition of the second link 112R. After completions of the acquisitions of the first and second links 112L, 112R, the master processing unit 120L responds by completing, e.g. terminating, the acquisition sequence of the exemplary binaural hearing system 125. The master processing unit 120L, the slave processing unit 120R, the first hearing implant 150L and second hearing implant 120R enter normal operation, i.e. a "payload mode", where the first and second ipsilateral data packets 455, 460, 405, 410, respectively, are exchanged in time slots 3 and 4 and the bilateral data packets 480, 430 are exchanged in time slots 1 and 2, respectively, of the consecutive frames as schematically illustrated on FIG. 6.

Some embodiments of the common communication protocol comprise that the master processing unit 120L is configured to respond to receipt of each of the acknowledge messages by starting a count-down sequence before each link acquisition is completed. The count-down sequence may comprises an exchange of several rounds, e.g. 2, 3 or 4 rounds, of the synchronization marker in question and the accompanying acknowledge message before termination of the link acquisition. The optional use of count-down sequences may further verify the reliability of the link in question.

The above outlined time-slot based scheme for synchronous transmission of the first and second ipsilateral data packets and bilateral data packets 480, 430 during normal operation of the exemplary binaural hearing system 125, as devised by the common communication protocol, leads to efficient use of the bandwidth of the respective wireless links. This time-slot based scheme for synchronous transmission is additionally energy efficient for the respective transceiver circuitries of the master processing unit 120L, the slave processing unit 120R, the first hearing implant 150L and second hearing implant 120R.

FIG. 7 illustrates an exemplary synchronization marker 465 that may be utilized for the respective acquisitions of the bilateral bidirectional wireless communication link 114, the first link 112L and the second link 112R. The exemplary synchronization marker 465 preferably comprises unique pair IDs 703 such as a unique number or code. A unique pair ID 703 may comprise between 8 bits and 32 bits. A first unique pair ID 703 may be utilized to pair the master hearing device 100L and the first hearing implant 150L to each other. The first unique pair ID 703 may be stored in a memory of the master processing unit 120L and further stored in a memory of the first control unit 108L of the first hearing implant 150L. The first unique pair ID 703 may for example be stored during manufacture of the master hearing device 100L or later in connection with a fitting of the binaural hearing system 125 to the user. The first unique pair ID 703 may be stored in a similar manner in the memory of the first control unit 108L. A second unique pair ID 703 may be utilized to pair the slave hearing device 100L and the second hearing implant 150L to each other. The second unique pair ID 703 may be stored in the memory of the master processing unit 120L and further stored in a memory of the second control unit 108R of the second hearing implant 150R. The second unique pair ID 703 may for example be stored in the master processing unit 120L and the second control unit 108R in a similar manner to the first unique pair ID 703 as outlined above.

A third unique pair ID 703 may be utilized to pair the slave hearing device 100L and the master hearing device 100L to each other. The third unique pair ID 703 may be stored in the memory of the master processing unit 120L and further stored in the memory of the slave processing unit 120R. The third unique pair ID 703 may for example be stored in the slave processing unit 120R and the master processing unit 120L in a similar manner to the first unique pair ID 703 as outlined above.

The use of unique pair IDs 703 ensures that only the appropriate pair of devices are connected to each other during the respective acquisitions of the first link 112L, second link 112R and bilateral link 114. Otherwise, unintended crosstalk between the devices, for example between the first and second second hearing implants 150L, 150R of the binaural hearing system 125 could lead to erroneous pairing of devices. The unique pair IDs 703 in a similar manner prevents unintended crosstalk and erroneous pairing of devices of two different but nearby located bilateral hearing systems.

The synchronization marker 465 can be viewed as special type of data packet and may have the same length and number of bits as at least some of the bilateral data packets. The synchronization marker 465 may comprise a predetermined search sequence 701 for example a predetermined binary pattern like 10101010 or equivalently 01010101. The latter is a priori known to both the master processing unit 120, the slave processing unit 120R and each of the first and second control units 108L, 108R for example by storage in respective memories of the units at manufacture. The predetermined binary pattern may be received and recognized by the slave processing unit 120R to detect the first synchronization marker 465c in a currently unoccupied time slot at the slave processing unit 120R. The predetermined binary pattern may likewise be received and recognized by the first control unit 108L of the first hearing implant 150L to detect the second synchronization marker 465a in a currently unoccupied time slot at the first control unit 108L. The predetermined binary pattern may likewise be received and recognized by the second control unit 108R of the second hearing implant 150R in a similar manner.

The synchronization marker 465 may comprise an optional wrap-around count-down number. The latter may be utilized by the master processing unit 120L and slave processing unit 120R for providing an additional layer of safety once the slave processing unit 120R and master processing unit 120L have acknowledged each other through the bilateral link as described in additional detail below. A slot indicator 709 tells the slave processing unit 120R in which time slot at the master side the synchronization marker 465 is arranged. This feature allows the slave processing unit 120R aligning its time slots with the corresponding time slots at the master processing unit 120L. The slave processing unit 120R may for example determine a time offset between already known time slots at the slave processing unit 120R and the corresponding time slots at the master processing unit 120R for alignment. Since, the length of a time slot, e.g. 48 µs, is known to the slave processing unit 120R at least via the common communication protocol the time offset can indicate slot alignment.

FIG. 8 illustrates an exemplary super-frame structure comprising where each super-frame comprises a plurality of individual frames, such as between 4 and 32 individual frames, of the plurality of consecutive frames in accordance with one embodiment of the common communication protocol. The frames at the master side of the binaural hearing system are structured in consecutive super-frames 901 and the super-frames at the slave side of the binaural hearing system are structured in consecutive super-frames 951. Each frame such as frame-1 may carry the same type of data for example digital audio signals which means that all data packets of the frame in question hold the same type of data e.g. digital audio signals, control information or error-correction codes like CRC and/or forward error-correction codes (FEC) etc. The super-frames at the master and slave sides may be misaligned in time, i.e. a time off-set, as schematically illustrated by an off-set arrow 953. The slave processing unit 120R may be configured to detect this time off-set and transmit that to the master processing unit 120L which proceeds to align the super-frames 901, 951 between the master side and slave side.

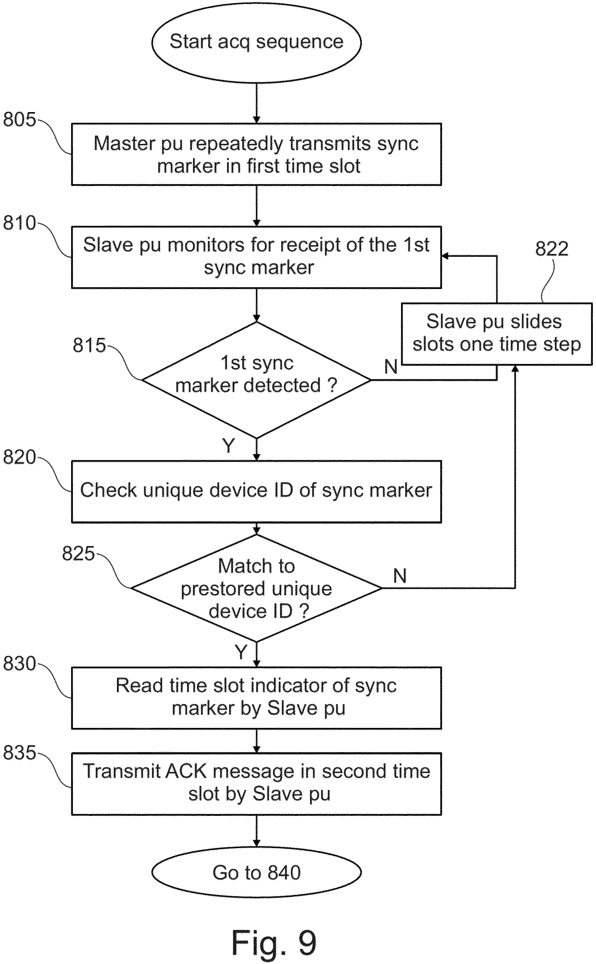

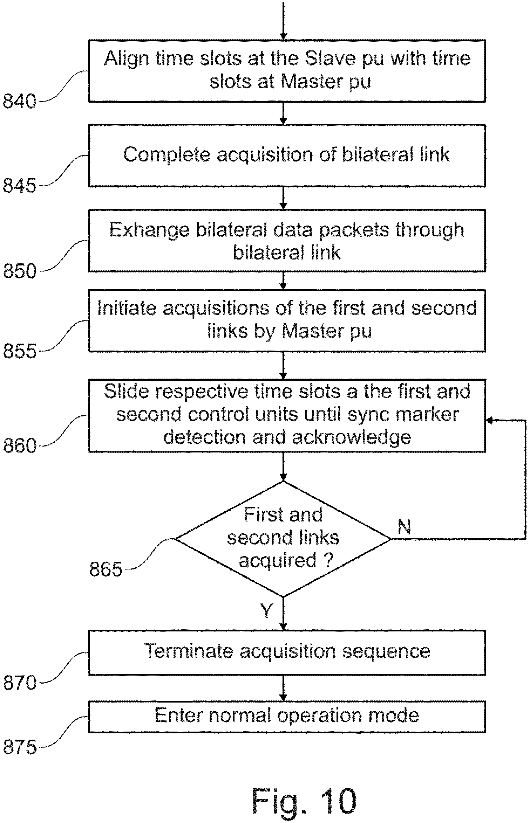

FIG. 9 and FIG. 10 are flow charts illustrating respective processing steps 805 - 875 carried out by the master and slave processing units of the first and second hearing devices in connection with the above-outlined acquisition sequence according to embodiments of the exemplary binaural hearing system 125.

## Claims

1. A binaural hearing system comprising:
a first hearing device, a second hearing device, a first hearing implant and a second hearing implant; wherein
the first hearing device is connectable to the first hearing implant via a first bidirectional wireless communication link for exchange of first ipsilateral data packets,
the second hearing device is connectable to the second hearing implant via a second bidirectional wireless communication link for exchange of second ipsilateral data packets,
a bilateral bidirectional wireless communication link is connectable between the first hearing device and second hearing device for exchange of bilateral data packets; wherein
said bilateral bidirectional wireless communication link and said first and second bidirectional wireless communication links are configured to operate in accordance with a common communication protocol which comprises a plurality of consecutive frames each comprising a plurality of time slots;
said common communication protocol comprising an acquisition sequence which comprises:
acquire the bilateral bidirectional wireless communication link using at least a first processing unit of the first hearing device and a second processing unit of the second hearing device,
acquire the first bidirectional wireless communication link in response to acquisition of the bilateral bidirectional wireless communication link using at least the first processing unit of the first hearing device,
acquire the second bidirectional wireless communication link in response to acquisition of the bilateral bidirectional wireless communication link using at least the second processing unit of the second hearing device.

2. A binaural hearing system according to claim 1, wherein the acquisition of the bilateral bidirectional wireless communication link comprises:
- transmit a first synchronization marker from the first processing unit to the second processing unit in a first time slot at the first processing unit,
- monitor the plurality of time slots at the second processing unit for the first synchronization marker,
- slide the plurality of time slots at the second processing unit with predetermined time steps from frame to frame of the plurality of consecutive frames using a wrap-around scheme,
- detect the first synchronization marker at the second processing unit,
- synchronize the plurality of time slots at the second processing unit with the plurality of time slots at the first processing unit based on the first synchronization marker,
- transmit an acknowledge message from the second processing unit to the first processing unit in a second time slot at the first processing unit,
- complete the acquisition of the bilateral bidirectional wireless communication link,
- start the exchange of the bilateral data packets through the bilateral bidirectional wireless communication link in the first and second times slots of the consecutive frames.

3. A binaural hearing system according to claim 1 or 2, wherein:
the acquisition of the first bidirectional wireless communication link comprises:
- transmit a second synchronization marker from the first processing unit to a first implant processor of the first hearing implant in a third time slot at the first processing unit,
- monitor the plurality of time slots at the first implant processor for receipt of the second synchronization marker,
- sliding the plurality of time slots at the first implant processor with predetermined time steps from frame to frame of the consecutive frames using a wrap-around scheme,
- detect the second synchronization marker at the first implant processor,
- synchronize the plurality of time slots at first implant processor with the plurality of time slots at the first processing unit based on the second synchronization marker,
- transmit an acknowledge message from the first implant processor to the first processing unit in a fourth time slot at the first processing unit,
- complete the acquisition of the first bidirectional wireless communication link.

4. A binaural hearing system according to claim 3, wherein: the acquisition of the second bidirectional wireless communication link comprises:
- transmit a third synchronization marker from the second processing unit to a second implant processor of the second hearing implant in a third time slot at the second processing unit,
- monitor the plurality of time slots, at the second implant processor for receipt of the third synchronization marker,
- sliding the plurality of time slots at the second implant processor with predetermined time steps from frame to frame of the plurality of consecutive frames using a wrap-around scheme,
- detect the third synchronization marker at the second implant processor,
- synchronize the plurality of time slots at second implant processor with the plurality of time slots at the second processing unit based on the third synchronization marker,
transmit an acknowledge message from the second implant processor to the second processing unit in a fourth time slot at the second processing unit,
- complete the acquisition of the second bidirectional wireless communication link; and
complete the acquisition sequence and enter normal operation of the binaural hearing system.

5. A binaural hearing system according to any of claims 2-4, wherein
- the first synchronization marker comprises a unique pair ID for pairing the first hearing device and the second hearing device; and
- the second synchronization marker comprises a unique pair ID for pairing the first hearing device and first hearing implant; and
- the third synchronization marker comprises a unique pair ID for pairing the second hearing device and the second hearing implant.

6. A binaural hearing system according to claim 4, wherein the second processing unit is configured to:
- compare the unique pair ID of the first synchronization marker with a pre-stored unique pair ID,
- ignore the first synchronization marker if the unique pair ID does not match the pre-stored unique pair ID; and
- transmit the acknowledge message if the pre-stored unique pair ID matches the unique pair ID of the first synchronization marker.

7. A binaural hearing system according to any of claims 2-6, wherein said common communication protocol comprises:
a plurality of super frames each comprising a plurality of individual frames, such as between 4 and 32 individual frames, of the plurality of consecutive frames.

8. A binaural hearing system according to any of claims 3-7, wherein each of the predetermined time steps for the sliding of the plurality of time slots is less than 5 % of a length of one time slot of the plurality of time slots of the frame.

9. A binaural hearing system according to any of claims 1-8, wherein the acquisition sequence comprises:
- maintain connection through the bilateral bidirectional wireless communication link during the acquisition of the first bidirectional wireless communication link and/or during the acquisition of the second bidirectional wireless communication link.

10. A binaural hearing system according to any of claims 4-9, wherein the second processing unit is configured to determine an offset in frames between consecutive super frames,
- transmit the offset to the first processing unit via the bilateral bidirectional wireless communication link,
- read the offset by the first processing unit,
- adjust timing of the super frames at the first processing unit to align super frames at the first and second processing units.

11. A binaural hearing system according to according to any of claims 2-10, wherein the synchronization marker at least comprises:
- a predetermined binary pattern, and/or
- a time slot indicator for indicating which time slot of the plurality of time slots at the first processing unit that holds the synchronization marker, and/or an error-detecting code.

12. A binaural hearing system according to any of claims 2-11, wherein each frame of the plurality of consecutive time frames comprises at least four non-overlapping time slots.

13. A binaural hearing system according to claim 12, wherein a length of each of the at least four non-overlapping time slots is identical.

14. A binaural hearing system according to any of the preceding claims, wherein at least a subset of the first and second ipsilateral data packets comprises respective digital audio data such as real-time digital audio signals; and/or at least a subset of the bilateral data packets comprises respective digital audio data such as real-time digital audio signals.

15. An acquisition sequence in accordance with a common communication protocol for acquiring respective connections between a first hearing device and a first hearing implant, a second hearing device and a second hearing implant and the first hearing device and the second hearing device;
said acquisition sequence comprising:
- acquire a bilateral bidirectional wireless communication link between the first hearing device and the second hearing device using a first processing unit of the first hearing device and a second processing unit of the second hearing device,
- exchange bilateral data packets between the first hearing device and the second hearing device,
- acquire a first bidirectional wireless communication link between the first hearing device and the first hearing implant, in response to acquisition of the bilateral bidirectional wireless communication link, using the first processing unit of the first hearing device,
- exchange first ipsilateral data packets between the first hearing device and the first hearing implant,
- acquire a second bidirectional wireless communication link between the second hearing device and second hearing implant, in response to acquisition of the bilateral bidirectional wireless communication link, using the second processing unit of the second hearing device
- exchange second ipsilateral data packets between the second hearing device and the second hearing implant;
- enter normal operation of the binaural hearing system.

16. An acquisition sequence according to claim 15, comprising:
- exchange the bilateral data packets in first and second time slots at the first processing unit of respective frames of the plurality of consecutive frames,
- exchange the first ipsilateral data packets in third and fourth time slots at the first processing unit of the respective frames of the plurality of consecutive frames,
- exchange the second ipsilateral data packets using in the third and fourth time slots at the second processing unit of respective frames of the plurality of consecutive frames.
